# EUROPÄISCHE PATENTSCHRIFT

(11) **EP 3 326 162 B1**
(45) Veröffentlichungstag und Bekanntmachung des Hinweises auf die Patenterteilung: **22.05.2019**
(21) Anmeldenummer: 16734196.5
(22) Anmeldetag: 01.07.2016
(51) Int. Cl.: G08B 25/01, A61B 5/00, G06F 19/00, G08B 7/06, G08B 25/10, G08B 25/00, H04W 76/00

(54) **VERFAHREN ZUR ALARMIERUNG VON PERSONEN**
PROCEDURE FOR ALERTING PEOPLE
PROCÉDÉ D'ALERTE DES PERSONNES

(30) Priorität: 17.07.2015 DE 102015009087
(43) Veröffentlichungstag der Anmeldung: 30.05.2018
(73) Patentinhaber: Drägerwerk AG & Co. KGaA, 23558 Lübeck (DE)
(72) Erfinder: BERNUTZ, Marco, 23617 Stockelsdorf (DE); LANDWEHR, Birger, 23569 Lübeck (DE)
(86) Internationale Anmeldenummer: PCT/EP2016/001131
(87) Internationale Veröffentlichungsnummer: WO 2017/012696

(56) Entgegenhaltungen:
- WO-A2-2004/070562
- US-A1- 2007 013 511
- US-A1- 2013 293 372

## Beschreibung

Die Erfindung betrifft ein Verfahren zur Alarmierung von Personen, die sich im Bereich eines Melders befinden, zum Beispiel von Pflegepersonal, gemäß Anspruch 1.

Derartige Verfahren dienen dazu, bei Auslösung eines Alarms zumindest eine Person zur Behebung des den Alarm auslösenden Ereignisses bereitzustellen, z.B. eine Pflegekraft zu einem Patienten zu rufen. Eine Herausforderung ist hierbei, möglichst schnell eine Person bereitzustellen, ohne dass die Personen durch ständige Alarme, die sie nicht wahrnehmen können oder bereits von anderen Personen erledigt werden, alarmmüde werden.

Aus US 7,439,856 B2 ist beispielsweise bekannt, mittels eines zentralen Alarmservers Personen nach einem Eskalationsstufenplan stufenweise mit Hilfe der Aktivierung eines Melders zu alarmieren, bis sich eine Person dem Alarmfall annimmt. Der Eskalationsstufenplan teilt die Personen nach Patientennamen, Patientenort, Krankheitszustand bzw. Alarmgrund des Patienten, der Verfügbarkeit weiterer Personen und deren Kombinationen ein. Es wird dann eine bestimmte Person oder Personenkreis zuerst alarmiert und anschließend stufenweise weniger gut in Frage kommende Personen alarmiert, wenn die vorangegangen alarmierten Personen den Alarmfall nicht annehmen.

Nachteilhaft hieran ist, dass die gesamte Eskalation von dem Alarmserver durchgeführt wird. Hierzu ist jedoch eine permanente Verbindung zu den Meldern notwendig, weil diese immer im Moment der Alarmierung durch den Server erreichbar sein müssen. Gerade Krankenhäuser sind jedoch weitläufig und teilweise auch gegen Strahlung abgeschirmt konstruiert. Es kommt daher häufig vor, dass der nächste Melder in der Eskalation gerade nicht erreichbar ist, wenn er alarmiert wird. Trotzdem wäre die zum Melder gehörige Person in diesem Moment eigentlich bereit dazu, sich des Alarmfalls anzunehmen. Wegen der nicht Erreichbarkeit wird jedoch die nächste Person in der Eskalation alarmiert, die den Alarmfall weniger effizient bearbeiten kann, zum Beispiel weil sie eine längere Wegstrecke zurücklegen muss oder schlechter qualifiziert ist.

Aufgabe der Erfindung ist es, Alarme möglichst zielgerichtet und zuverlässig an verantwortliche Personen, z. B. Pflegekräfte zu leiten und somit eine hohe Effizienz in der Alarmfallbearbeitung zu erreichen und einer Alarmmüdigkeit entgegen zu wirken.

Hauptmerkmale der Erfindung sind in Anspruch 1 angegeben. Ausgestaltungen sind Gegenstand der Unteransprüche.

Die Erfindung betrifft daher ein Verfahren zur Alarmierung von Personen, die sich im Bereich eines Melders befinden, in Abhängigkeit von Anforderungssignalen von wenigstens einer Alarmquelle, wobei jeder Melder (insbesondere eigenständig) aktuelle Countdowns für jede der Alarmquellen festlegt. Bei Ausgabe eines Anforderungssignals von einer der Alarmquellen werden die zugehörigen Countdowns aller Melder gestartet, die das Anforderungssignal empfangen. Der Melder, dessen gestarteter Countdown abläuft, gibt ein Alarmsignal aus. Die manuelle Annahme eines ausgegebenen Alarmsignals durch eine Person, die sich im Bereich des Melders befindet, der das Alarmsignal ausgibt, bricht die gestarteten Countdowns der anderen Melder ab, welche dadurch insbesondere zurück auf Start gesetzt werden.

Damit werden Alarmsignale nicht von allen möglichen Meldern gleichzeitig signalisiert, sondern gemäß den Countdownlängen verzögert und zeitlich gestaffelt eskaliert, insbesondere bis sich eine Person dem Fall annimmt. Auf diese Weise erhalten die Personen insgesamt weniger Alarmsignale, wodurch eine Alarmmüdigkeit reduziert wird. Um eine Person auf das Alarmsignal aufmerksam zu machen, sollte es sich insbesondere aber nicht ausschließlich um ein akustisches und/oder visuelles und/oder taktiles Signal handeln. Weiterhin können Alarmsignale Textnachrichten sowie Kontextinformationen umfassen, die dem Anwender angezeigt werden.

Erreichbarkeit wird jedoch die nächste Person in der Eskalation alarmiert, die den Alarmfall weniger effizient bearbeiten kann, zum Beispiel weil sie eine längere Wegstrecke zurücklegen muss oder schlechter qualifiziert ist.

Im Übrigen ist aus der WO 2004/070562 A2 ein Verfahren zur Alarmierung von Klinikpersonal, das sich im Bereich eines Alarmmelders befindet, in Abhängigkeit spezieller Anforderungssignale bekannt. Nach Übertragung des Alarmsignals auf den Alarmmelder wird zunächst ein Countdown gestartet. Sollte der Countdown ablaufen bevor der Empfang von der alarmierten Person bestätigt wurde, wird der Alarm eskaliert, insbesondere an einen weiteren Alarmmelder übertragen.

Ferner beschreibt die US 2013/0293372 A1 ein Alarmsystem, bei dem im Alarmfall ein Countdown auf einem zentralen Server gestartet wird und dieser erst beendet wird, sobald der Erhalt von wenigstens einem Empfänger bestätigt wurde.

Aufgabe der Erfindung ist es, Alarme möglichst zielgerichtet und zuverlässig an verantwortliche Personen, z. B. Pflegekräfte zu leiten und somit eine hohe Effizienz in der Alarmfallbearbeitung zu erreichen und einer Alarmmüdigkeit entgegen zu wirken.

Hauptmerkmale der Erfindung sind in Anspruch 1 angegeben. Ausgestaltungen sind Gegenstand der Unteransprüche.

Die Erfindung betrifft daher ein Verfahren zur Alarmierung von Personen, die sich im Bereich eines Melders befinden, in Abhängigkeit von Anforderungssignalen von wenigstens einer Alarmquelle, wobei jeder Melder (insbesondere eigenständig) aktuelle Countdowns für jede der Alarmquellen festlegt. Bei Ausgabe eines Anforderungssignals von einer der Alarmquellen werden die zugehörigen Countdowns aller Melder gestartet, die das Anforderungssignal empfangen. Der Melder, dessen gestarteter Countdown abläuft, gibt ein Alarmsignal aus. Die manuelle Annahme eines ausgegebenen Alarmsignals durch eine Person, die sich im Bereich des Melders befindet, der das Alarmsignal ausgibt, bricht die gestarteten Countdowns der anderen Melder ab, welche dadurch insbesondere zurück auf Start gesetzt werden.

Damit werden Alarmsignale nicht von allen möglichen Meldern gleichzeitig signalisiert, sondern gemäß den Countdownlängen verzögert und zeitlich gestaffelt eskaliert, insbesondere bis sich eine Person dem Fall annimmt. Auf diese Weise erhalten die Personen insgesamt weniger Alarmsignale, wodurch eine Alarmmüdigkeit reduziert wird. Um eine Person auf das Alarmsignal aufmerksam zu machen, sollte es sich insbesondere aber nicht ausschließlich um ein akustisches und/oder visuelles und/oder taktiles Signal handeln. Weiterhin können Alarmsignale Textnachrichten sowie Kontextinformationen umfassen, die dem Anwender angezeigt werden.

Eine Koordination der Eskalation seitens der Alarmquelle ist nicht erforderlich. Die Eskalation steht im Moment der Ausgabe des Anforderungssignals aufgrund der Erkennung einer Alarmbedingung fest und wird vor allem von den Meldern selbst abgearbeitet. Es ist nicht einmal notwendig, dass die Melder den Beginn des Countdowns oder den Erhalt des Anforderungssignals bestätigen. Es entsteht eine hohe Kompatibilität mit unterschiedlichen Alarmquellen, ohne dass ein zentraler Alarmserver erforderlich wäre.

Ein weiterer Vorteil ist, dass die erfindungsgemäßen Countdowns auch dann weiterlaufen, wenn sich der Melder in ein Funkloch bewegt, in dem er kurzzeitig nicht erreichbar ist. Die Person kann hierdurch in diesem Moment trotz fehlendem Funkkontakt durch den Melder alarmiert werden. Selbst wenn die manuelle Annahme kurzeitig nicht ausgesendet werden kann, geschieht dies meist noch während die anderen Countdowns laufen, weil sich die Person mit dem Melder in Bewegung setzen wird, um sich dem Alarmfall anzunehmen.

Umgekehrt müssen auch beim Start der Countdowns nicht zwingend alle Melder unmittelbar das Anforderungssignal empfangen. Es genügt, wenn das Anforderungssignal in einem definierten Zeitraum empfangen wird. Erst wenn ein Melder länger als der definierte Zeitraum keine optionale Bestätigung ausgibt, sollte er vorzugsweise aus dem Eskalationsplan fallen und die noch laufenden längeren Countdowns verkürzt werden. Wenn eine Person am Melder ein Alarmsignal bestätigt hat, ist diese Bestätigung vorzugweise zeitlich begrenzt. Hierdurch kann sichergestellt werden, dass die Alarmquelle nach einer bestimmten Zeit selbständig ein eigenes Alarmsignal ausgibt, wenn die benachrichtigte Person nicht oder nur verzögert zur Alarmquelle bzw. zum Patienten kommt und die Ursache behebt.

Zur Festlegung der Countdowns durch die Melder gehört auch, eine Sperrung des Countdowns gegen ein Starten oder alternativ ein Heraufsetzen des Countdowns auf den Wert unendlich, wenn der Melder nicht an der Eskalation einer Alarmquelle teilnehmen soll, zum Beispiel aufgrund eines zu großen Abstands oder wegen mangelnder Qualifikation der zum Melder gehörigen Person. Gemäß einer näheren Ausgestaltung des Verfahrens ist vorgesehen, dass wenigstens einer der Melder ein tragbarer Funkmelder ist, welchen eine der Personen mit sich führt. Damit ist das Verfahren auch dazu geeignet mobil agierende Personen zu alarmieren. Die tragbaren Funkmelder sind insbesondere drahtlos mit der oder den Alarmquellen verbunden, insbesondere mittelbar oder unmittelbar.

Umgekehrt besteht auch die Option, dass das Verfahren dahingehend ergänzt ist, dass wenigstens einer der Melder ein stationärer Melder ist, welcher Personen in der örtlichen Umgebung alarmiert. Damit können beispielsweise Personen in Bereitschaftsräumen oder am Empfang alarmiert werden.

Nach einer spezifischen Ausgestaltung des Verfahrens reduziert die manuelle Ablehnung eines Alarmsignals durch eine Person, die sich im Bereich des Melders befindet, der ein Alarmsignal ausgibt, den Countdown der anderen Melder, vorzugsweise um die Zeitspanne des noch laufenden kürzesten Countdowns. Bei Ablehnung eines Alarms wird demnach schneller bzw. sofort der nächst Melder aktiviert und gibt ein Alarmsignal aus.

Weil die Ausgabe eines Anforderungssignals an der Alarmquelle meist einen unmittelbaren Handlungsbedarf kennzeichnet, sieht eine Ergänzung des Verfahrens vor, dass der kürzeste Countdown für jede Alarmquelle gleich null ist und der oder die diesen kürzesten Countdown enthaltenden Melder sofort ein Alarmsignal ausgeben, wenn sie ein Anforderungssignal empfangen.

Die Vorteile des Verfahrens werden besonders deutlich in Varianten, in denen wenigstens zwei Alarmquellen vorhanden sind und jedem ausgegebenen Anforderungssignal eine Kennung der auslösenden Alarmquelle zugefügt ist. Für jede Alarmquelle organisieren die Melder dann in Kleingruppen die Countdownlängen der Eskalation. Hierzu tauschen sie mittel- oder unmittelbar Informationen untereinander innerhalb der Kleingruppen aus. Auf diese Weise können mehrere Alarmquellen unabhängig voneinander Personen anfordern. Die Eskalationen laufen dabei unabhängig nach den jeweiligen Countdownlängen ab. Als Alarmquellen kommen insbesondere Patientenklingeln, mobile Notrufeinrichtung (auch Mobiltelefone), Ventilatoren, Anästhesiegeräte, Monitore, Spritzenpumpen oder Software, z.B. auf einem Alarmserver, in Betracht.

Eine nähere Ausführungsform des Verfahrens sieht vor, dass die Alarmquellen und die Melder jeweils ausschließlich mit einem Alarmmanagement-Server kommunizieren. Hierdurch kann die Informationsinfrastruktur vereinfacht werden. Außerdem ist es möglich auch Fremdgeräte einzubinden, z.B. Mobiltelefone. Insbesondere wird mit dem Server eine zentrale Kontrolleinrichtung geschaffen. Mit dem Server lassen sich unterschiedliche Geräte durch eine zentrale Schnittstelle einbinden. Vom Server können dann unterschiedliche Datentransferprotokolle der Melder und Alarmquellen konvertiert werden, wodurch eine Kompatibilität entsteht. Außerdem sind Parameter für die Länge der Countdowns zentral einstellbar und müssen nicht an den Alarmquellen eingestellt werden. Die Alarmquelle muss damit nur dazu geeignet sein, überhaupt ein Anforderungssignal auszugeben. Die Systemanforderungen zur Erfassung der Parameter werden zentral erfüllt. Am Server kann auch eine Schulung einfacher durchgeführt werden als beispielsweise in Krankenzimmern. Zusätzlich sind Zugangsberechtigungen zu den Servern festlegbar.

Darüber hinaus bieten Alarmmanagement-Server folgende vorteilhafte Möglichkeiten:
- Interpretation der von der Alarmquelle eintreffenden Anforderungssignale und Vitaldaten des Patienten, um
   ∘ die Weiterleitung der Anforderungssignale an die Melder zu priorisieren,
   ∘ neue (Multi-Parameter-)Alarmsignale zu erzeugen und diese weiterzuleiten,
   ∘ die Weiterleitung der Anforderungssignale an Melder zu unterdrücken,
   ∘ einzelne Anforderungssignale durch neue Anforderungssignale (und deren Zustände, z.B. "Niedriger Atemwegsdruck" wird zu "Patient vom Beatmungsgerät diskonnektiert") zu ersetzen,
   ∘ Alarmsignale zu konsolidieren,
      ▪ indem aufgrund von Kontextwissen mehrere Anforderungssignale zu einem einzelnen zusammengefasst werden,
      ▪ indem Alarme, die von derselben Alarmquelle kommen, derselben Person zugeordnet werden, z.B. wenn eine Person ein Alarmsignal bestätigt hat und noch weitere Anforderungssignale von derselben Alarmquelle kommen, sollen keine anderen Personen mehr alarmiert werden.
- Anforderungssignale, die von derselben Alarmquelle kommen, sollen i.d.R. derselben Person zugeordnet werden, soweit die Person hierfür verantwortlich ist.

Eine besondere Ausgestaltungsvariante des Verfahrens sieht vor, dass von jedem Melder die Position relativ zu jeder der Alarmquellen ermittelt und den anderen Meldern bereitgestellt wird, wobei die Länge der Countdowns von der eigenen Position und den Positionen der anderen Melder relativ zu der Alarmquelle abhängt, zu der der Countdown festgelegt wird. Damit lassen sich an bestimmten Orten befindliche Personen zuerst alarmieren. Bei stationären Meldern ist dies durch feste, insbesondere einmalige, Positionsangabe realisierbar. Bei mobilen Meldern ist dies beispielsweise durch Feldstärkeermittlungen in WLAN-Netzwerken, RFID-Informationen, Barcodescanner, QR-Codes oder Nutzereingaben realisierbar.

Gemäß einer weiteren näheren Ausgestaltung legt jeder Melder seine Countdowns unter Berücksichtigung der absoluten Entfernungen oder räumlichen Wegentfernung des Melders und den anderen Meldern zum Einsatzort der zugehörigen Alarmquelle fest. Damit lassen sich Personen zuerst alarmieren, deren Wegstrecke zum Einsatzort kurz ist.

Des Weiteren ist eine Verfahrensvariante vorgesehen, nach der jeder Countdown in Echtzeit oder getaktet aktualisiert wird. Damit ist eine Balance zwischen möglichst exakter Personenalarmierung und zum Beispiel Akkulaufzeiten erzielbar, weil insbesondere für den Datentransfer Stromverbrauch stattfindet. Umgesetzt werden kann die Aktualisierung der Countdowns beispielsweise indem ein Aufforderungssignal an die Melder ausgegeben wird, auf welches hin sich die das Aufforderungssignal empfangenden Melder zurückmelden, insbesondere durch Rücksendung eines Verfügbarkeitssignals und/oder durch Rücksendung eines Positionssignals.

Bei einer Verfahrensmodifikation wird jeder Countdown unter Berücksichtigung des Melders, insbesondere unter Berücksichtigung der dem Melder zugeordneten Personen, festgelegt. Damit können beispielsweise besser qualifizierte Personen vorrangig alarmiert werden, oder aber zuerst ein Alarmsignal im Personalzimmer und erst anschließend bei den mobilen Meldern ausgelöst werden.

Ergänzt werden kann das Verfahren dadurch, dass durch eine Person eine fehlende Verfügbarkeit am Melder eingebbar ist, die bei der Festlegung, insbesondere bei der Aktualisierung, eines jeden Countdowns berücksichtigt wird. Die nicht verfügbare Person wird dann nicht durch ein Alarmsignal gestört und die Eskalation der Countdowns nicht durch ohnehin nicht verfügbare Personen verlangsamt.

Vergleichbare Vorteile werden erzielt, wenn ein Melder nach Annahme eines Alarmsignals für einen definierten Zeitraum als nicht verfügbar gilt, was bei der Festlegung, insbesondere bei der Aktualisierung, eines jeden Countdowns berücksichtigt wird. Eine im Einsatz befindliche Person wird dann nicht durch ein weiteres Alarmsignal gestört. Umsetzbar ist dies entweder durch nicht-starten des Countdowns auf dem Melder oder zumindest durch Verlängerung des Countdowns auf Werte, die größer sind als die Countdowns der anderen Melder.

Darüber hinaus sieht eine Abwandlung des Verfahrens vor, dass die Alarmquelle bei Ausgabe eines Anforderungssignals einen Sicherheitscountdown startet, der länger ist als die mit dem Anforderungssignal gestarteten Countdowns der Melder, wobei bei Ablauf des Sicherheitscountdowns von der Alarmquelle wieder ein Anforderungssignal ausgegeben wird oder ein eigenes akustisches und/oder visuelles und/oder taktiles Alarmsignal ausgegeben wird. Damit wird verhindert, dass sich keine Person dem Alarmfall annimmt.

Ferner ist eine Erweiterung des Verfahrens von Vorteil, bei dem festlegbar ist, welche der Melder welcher der Alarmquellen zugeordnet sind, und dies bei der Festlegung der Countdowns berücksichtigt wird. Damit kann festgelegt werden, welche Personen geeignet sind, um sich einem Alarmfall anzunehmen. Beispielsweise kann für die Alarmquellen festgelegt werden, welche Person qualifiziert und welche nicht qualifiziert ist.

Weil der Einsatz bei jeder Alarmquelle unterschiedliche Anforderungen hat, ist es vorteilhaft, wenn die Countdowns hinsichtlich ihrer Länge für jede Alarmquelle individuell festlegbar und/oder festgelegt sind.

Über eine weitere Abwandlung des Verfahrens lassen sich Strategien zur Optimierung der Auslastung und auch Entlastung des klinischen Personals umsetzen. Insbesondere kann durch eine Analyse, wie häufig eine Person im Vergleich zu den anderen Personen Alarmsignale angenommen hat, eine Umverteilung der Anforderungssignale auf alle Melder durchgeführt werden. Hierbei werden bei jedem Melder die eingehenden Anforderungssignale der am Melder ausgelösten Alarmsignale sowie die von der entsprechenden Person bestätigten Alarmsignale gespeichert und ausgewertet. Aus der Bewertung werden die Countdowns der Personen mit wenigen Annahmen entsprechend verkürzt oder die der häufig annehmenden Personen verlängert.

Eine spezielle Ausführung sieht vor, dass die Countdowns von wenigstens zwei Meldern sowie für die gleiche Alarmquelle gleich groß sind. Damit können zwei Personen gleichzeitig angefordert werden. Dies ist besonders hilfreich bei erforderlicher Teamversorgung oder besonders hoher Dringlichkeit eines Alarms. Im Krankenhausbereich, sollte der kürzeste Countdown so kurz wie möglich und der längste Countdown so lange wie klinisch verantwortbar sein.

Weitere Merkmale, Einzelheiten und Vorteile der Erfindung ergeben sich aus dem Wortlaut der Ansprüche sowie aus der folgenden Beschreibung eines Ausführungsbeispiels anhand einer Zeichnung. Es zeigt:

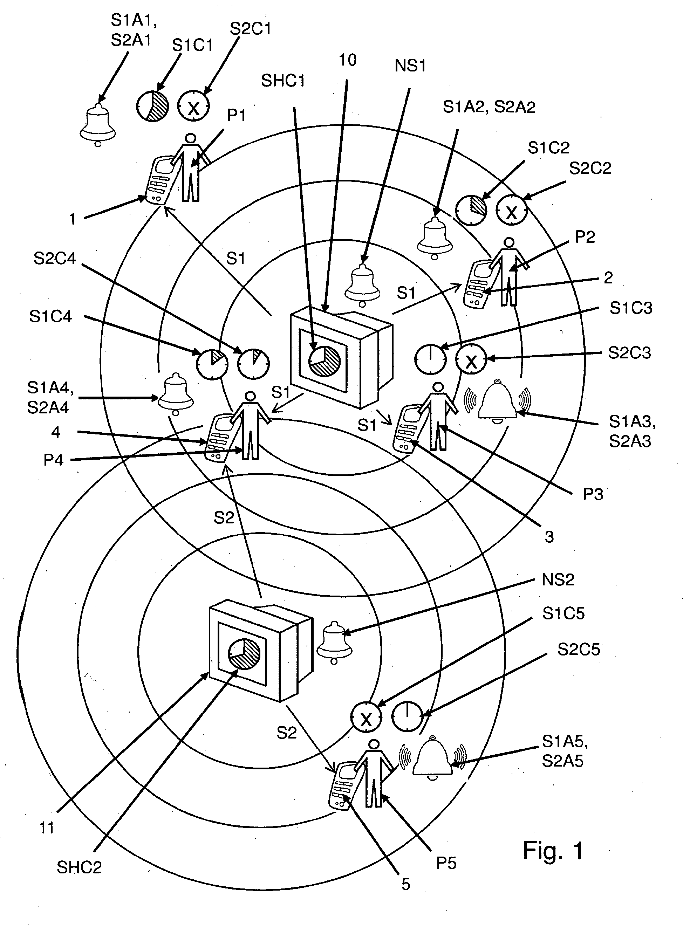

- Fig. 1: eine schematische Darstellung von Personen mit Meldern im Umfeld von zwei Alarmquellen.

In Fig. 1 sieht man eine schematische Darstellung von fünf Personen P1, P2, P3, P4, P5, die jeweils einen Melder 1, 2, 3, 4, 5 mit sich führen, im Umfeld von zwei Alarmquellen 10, 11. Bei den Meldern 1, 2, 3, 4, 5 handelt es sich um tragbare Funkmelder, die drahtlos mit den Alarmquellen 10, 11 verbunden sind. Jeweils drei Ringe um die Alarmquellen 10, 11 symbolisieren eine Einstufung der Melder 1, 2, 3, 4, 5 nach dem Wegabstand von diesen. Personen P1, P2, P3, P4, P5 außerhalb der Ringe sind zu weit von der jeweiligen Alarmquelle 10, 11 entfernt. Sie stehen für eine Annahme eines Anforderungssignals S1, S2 nicht zur Verfügung.

Weiterhin sieht man, dass jeder Person zwei Uhren mit jeweils einem Countdown S1C1, S1C2, S1C3, S1C4, S1C5, S2C1, S2C2, S2C3, S2C4, S2C5 zugeordnet sind. Diese werden von den Meldern 1, 2, 3, 4, 5 gestartet, wenn sie wie dargestellt ein Anforderungssignal S1, S2 erhalten. Man erkennt, dass
- der erste Melder 1 ein erstes Anforderungssignal S1 empfangen hat und ein erster Countdown S1C1 läuft. Sollte der erste Countdown S1C1 ablaufen, würde ein Alarmsignal S1A1 ausgelöst. Der erste Melder 1 liegt außerhalb des Zuständigkeitsradius der zweiten Alarmquelle 11, sodass kein zweiter Countdown S2C1 gestartet wurde. Damit kann in der gezeigten Position der ersten Person P1 auch kein potentielles Alarmsignal S2A1 ausgelöst werden.
- der zweite Melder 2 ein erstes Anforderungssignal S1 empfangen hat und ein erster Countdown S1C2 läuft. Sollte der erste Countdown S1C2 ablaufen, würde ein Alarmsignal S1A2 ausgelöst. Der zweite Melder 2 liegt außerhalb des Zuständigkeitsradius der zweiten Alarmquelle 11, sodass kein zweiter Countdown S2C2gestartet wurde. Damit kann in der gezeigten Position der zweiten Person P2 auch kein potentielles Alarmsignal S2A2 ausgelöst werden.
- der dritte Melder 3 ein erstes Anforderungssignal S1 empfangen hat und ein erster Countdown S1C3 abgelaufen ist. Der dritte Melder 3 liegt jedoch außerhalb des Zuständigkeitsradius der zweiten Alarmquelle 11, sodass kein zweiter Countdown S2C3 gestartet wurde. Der abgelaufene erste Countdown S1C3 hat ein Alarmsignal S1A3 ausgelöst, welcher nunmehr von der dritten Person P3 angenommen oder abgelehnt werden kann. Dem Alarmsignal S1A3 ist eine Kennung beigefügt, damit es von der dritten Person 3 von einem potentiellen Alarmsignal S2A3 unterscheiden kann, welches vom zweiten Countdown S2C3 auslösbar wäre.
- der fünfte Melder 5 ein zweites Anforderungssignal S2 empfangen hat und daraufhin ein zweiter Countdown S2C5 abgelaufen ist, welcher ein Alarmsignal S2A5 ausgelöst hat. Der fünfte Melder 5 liegt außerhalb des Zuständigkeitsradius der ersten Alarmquelle 10, sodass kein erster Countdown S1C5 gestartet wurde. Damit kann in der gezeigten Position der ersten Person P5 auch kein potentielles Alarmsignal S1A5 ausgelöst werden.
- der vierte Melder 4 ein erstes Anforderungssignal S1 und ein zweites Anforderungssignal S2 empfangen hat und ein erster Countdown S1C4 sowie ein zweiter Countdown S2C4 laufen. Sollte der erste Countdown S1C4 ablaufen, würde ein Alarmsignal S1A4 ausgelöst. Sollte der zweite Countdown S2C4 ablaufen, welcher schon weiter fortgeschritten ist, wird jedoch zunächst ein Alarmsignal S2A4 ausgelöst. Sollte die dritte Person P3 ihr Alarmsignal S1A3 annehmen wird der erste Countdown S1C4 abgebrochen. Nimmt die fünfte Person P5 das ausgelöste Alarmsignal S2A5 an, bricht der zweite Countdown S2C4 ab.

Durch das erste Anforderungssignal S1 werden also in chronologischer Reihenfolge Alarmsignale S1A3, S1A4, S1A2, S1A1 bei der dritten Person P3, dann bei der vierten Person P4, dann bei der zweiten Person P2 und als letztes bei der ersten Person P1 ausgelöst. Zur Sicherheit läuft bei der ersten P Alarmquelle 10 ein erster Sicherheitscountdown SHC1 mit. Dieser ist länger als die ersten Countdowns S1C1, S1C2, S1C3, S1C4 und bei Ablauf wird erneut ein erstes Anforderungssignal S1 ausgegeben, um alle verfügbaren Melder 1, 2, 3, 4, 5, die dann im Verfügbarkeitsbereich der ersten Alarmquelle 10 sind, zu erreichen und eine neue Eskalation durch Starten der ersten Countdowns S1C1, S1C2, S1C3, S1C4, S1C5 zu starten. Zusätzlich oder alternativ kann die erste Alarmquelle 10 optional ein eigenes akustisches, visuelles und/oder taktiles Alarmsignal NS1 ausgeben.

Hinsichtlich der zweiten Alarmquelle 11 werden in chronologischer Reihenfolge Alarmsignale S2A5, S2A4 bei der fünften Person P5 und dann bei der vierten Person P4 ausgegeben. Zur Sicherheit läuft auch bei der zweiten Alarmquelle 11 ein Sicherheitscountdown SHC2 mit. Bei dessen Ablauf gibt die zweite Alarmquelle 11 erneut ein zweites Anforderungssignal S2 aus, um alle verfügbaren Melder 1, 2, 3, 4, 5, die dann im Verfügbarkeitsbereich der zweiten Alarmquelle 11 sind, zu erreichen und eine neue Eskalation durch Starten der zweiten Countdowns S1C1, S1C2, S1C3, S1C4, S1C5 zu starten. Zusätzlich oder alternativ kann die zweite Alarmquelle 11 optional ein eigenes akustisches, visuelles und/oder taktiles Alarmsignal NS2 ausgeben.

Mit diesen Gegebenheiten lässt sich also ein Verfahren zur Alarmierung der Personen P1, P2, P3, P4, P5, die sich im Bereich der Melder 1, 2, 3, 4, 5 befinden, in Abhängigkeit von den Anforderungssignalen S1, S2 von den zwei Alarmquellen 10, 11 durchführen, bei dem jeder Melder 1, 2, 3, 4, 5 aktuelle Countdowns S1C1, S1C2, S1C3, S1C4, S1C5, S2C1, S2C2, S2C3, S2C4, S2C5 für jede der Alarmquellen 10, 11 festlegt. Dabei haben der erste, zweite und dritte Melder 1, 2, 3 den zweiten Countdown S2C1, S2C2, S2C3 jeweils gesperrt, weil sie zu weit weg von der zweiten Alarmquelle 11 sind. Entweder stellen die Melder 1, 2, 3, 4, 5 die Position außerhalb der Zone um die Alarmquelle 10, 11 selbst fest oder die Melder 1, 2, 3, 4, 5 erhalten innerhalb der Zone ein Aufforderungssignal zum Festlegen des Countdowns S1C1, S1C2, S1C3, S1C4, S1C5, S2C1, S2C2, S2C3, S2C4, S2C5 sowie außerhalb der Zone keines.

Zur Festlegung der Countdowns S1C1, S1C2, S1C3, S1C4, S1C5, S2C1, S2C2, S2C3, S2C4, S2C5 sollte von jedem Melder 1, 2, 3, 4, 5 die eigene Position relativ zu jeder der Alarmquellen 10, 11 ermittelt und den anderen Meldern 1, 2, 3, 4, 5 bereitgestellt werden. Die Länge der Countdowns S1C1, S1C2, S1C3, S1C4, S1C5, S2C1, S2C2, S2C3, S2C4, S2C5 wird dann in Abhängigkeit von der eigenen Position und den Positionen der anderen Melder 1, 2, 3, 4, 5 relativ zu der Alarmquelle 10, 11 festgelegt.

Insbesondere sollte jeder Melder 1, 2, 3, 4, 5 seine Countdowns S1C1, S1C2, S1C3, S1C4, S1C5, S2C1, S2C2, S2C3, S2C4, S2C5 unter Berücksichtigung der absoluten Entfernungen oder räumlichen Wegentfernung des Melders 1, 2, 3, 4, 5 und den anderen Meldern 1, 2, 3, 4, 5 zum Einsatzort der zugehörigen Alarmquelle 10, 11 festlegen.

Als weitere Parameter zur Festlegung der Countdowns S1C1, S1C2, S1C3, S1C4, S1C5, S2C1, S2C2, S2C3, S2C4, S2C5 kommt die dem Melder 1, 2, 3, 4, 5 zugeordneten Person P1, P2, P3, P4, P5 in Betracht, um deren Qualifikation, Zuständigkeit und Funktion angemessen bei der Eskalation zu berücksichtigen. Jeder Countdown S1C1, S1C2, S1C3, S1C4, S1C5, S2C1, S2C2, S2C3, S2C4, S2C5 kann in Echtzeit oder getaktet aktualisiert werden.
Des Weiteren besteht die Option, dass durch eine Person P1, P2, P3, P4, P5 eine fehlende Verfügbarkeit am Melder 1, 2, 3, 4, 5 eingebbar ist, die bei der Festlegung, insbesondere bei der Aktualisierung, eines jeden Countdowns S1C1, S1C2, S1C3, S1C4, S1C5, S2C1, S2C2, S2C3, S2C4, S2C5 berücksichtigt wird. Dies kann auch eine generelle nicht Verfügbarkeit bestimmter Personen P1, P2, P3, P4, P5 für bestimmte Aufgaben bzw. Alarmquellen 10, 11 umfassen.

Ferner kann ein Melder 1, 2, 3, 4, 5 nach Annahme eines Alarmsignals S1A1, S1A2, S1A3, S1A4, S1A5, S2A1, S2A2, S2A3, S2A4, S2A5 für einen definierten Zeitraum als nicht verfügbar gelten, was bei der Festlegung, insbesondere bei der Aktualisierung, eines jeden Countdowns S1C1, S1C2, S1C3, S1C4, S1C5, S2C1, S2C2, S2C3, S2C4, S2C5 berücksichtigt wird.

Bei der gezeigten Ausgabe eines Anforderungssignals S1, S2 von einer der Alarmquellen 10, 11 werden die zugehörigen Countdowns S1C1, S1C2, S1C3, S1C4, S1C5, S2C1, S2C2, S2C3, S2C4, S2C5 aller Melder 1, 2, 3, 4, 5, 5 gestartet, die das Anforderungssignal S1, S2 empfangen. Der Melder 1, 2, 3, 4, 5, dessen gestarteter Countdown S1C1, S1C2, S1C3, S1C4, S1C5, S2C1, S2C2, S2C3, S2C4, S2C5 abläuft, gibt ein Alarmsignal S1A1, S1A2, S1A3, S1A4, S1A5, S2A1, S2A2, S2A3, S2A4, S2A5 aus.

Bei manueller Annahme eines ausgegebenen Alarmsignals S1A1, S1A2, S1A3, S1A4, S1A5, S2A1, S2A2, S2A3, S2A4, S2A5 durch die Person P1, P2, P3, P4, P5, die sich im Bereich des Melders 1, 2, 3, 4, 5 befindet, der das Alarmsignal S1A1, S1A2, S1A3, S1A4, S1A5, S2A1, S2A2, S2A3, S2A4, S2A5 ausgibt, werden die gestarteten Countdowns S1C1, S1C2, S1C3, S1C4, S1C5, S2C1, S2C2, S2C3, S2C4, S2C5 der anderen Melder 1, 2, 3, 4, 5 abgebrochen und zurück auf Start gesetzt.

Wenn jetzt beispielsweise die dritte oder fünfte Person P3, P5 die ausgelösten Alarmsignale S1A3, S2A5 manuell ablehnt, können die noch laufenden Countdowns S1C1, S1C2, S1C4, S2C4 der anderen Melder 1, 2, 4 reduziert werden, nämlich insbesondere jeweils um die Zeitspanne des noch laufenden kürzesten Countdowns S1C4, S2C4.

Nicht zu erkennen ist, ob in Fig. 1 der kürzeste Countdown S1C3, S2C5 für jede Alarmquelle 10, 11 bereits beim Start den Wert null aufwies, sodass die die kürzesten Countdowns S1C3, S2C5 enthaltenden Melder 3, 5 sofort das Alarmsignal S1A3, S2A5 ausgeben haben, als sie das Anforderungssignal S1, S2 empfingen. Dies ist jedoch optional vorsehbar.

Bei den Alarmsignalen S1A3, S2A5 kann es sich um akustische und/oder visuelle und/oder taktile Signale handeln. Insbesondere enthalten diese auch eine Information darüber, zu welcher der Alarmquellen die ausgelösten Alarmsignalen S1A3, S2A5 gehören. Bevorzugt erfolgt dies über eine Anzeige. Die Anzeige kann optional auch darstellen,
- wie viele Melder noch in dem Eskalationsplan einer Primäralarmquelle 10, 11 enthalten sind, auf dem ersten Melder 1 zum Beispiel durch Angabe 10: 4; 11: 2.
- welche Melder noch in dem Eskalationsplan einer Primäralarmquelle 10, 11 enthalten sind, auf dem ersten Melder 1 zum Beispiel durch Angabe 10: 1, 2, 3, 4; 11: 4, 5.
- in welcher Reihenfolge die Eskalation der Melder in dem Eskalationsplan einer Primäralarmquelle 10, 11 angeordnet sind, auf dem ersten Melder 1 zum Beispiel durch Angabe 10: 3, 4, 2, 1; 11: 5, 4.
- wie der Status der anderen Melder 1, 2, 3, 4, 5 ist, auf dem ersten Melder 1 zum Beispiel durch Angabe 10: ((3)), 4, 2, 1, x5x; 11: ((5)), 4, x1x, x2x, x3x; Die Klammern symbolisieren eine Alarmsignalausgabe, die Kreuze x eine fehlende Verfügbarkeit.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### BEZUGSZEICHENLISTE

- 1: erster Melder
- 2: zweiter Melder
- 3: dritter Melder
- 4: vierter Melder
- 5: fünfter Melder

- 10: erste Alarmquelle
- 11: zweite Alarmquelle

- NS1: Alarmsignal (Alarmquelle)
- NS2: Alarmsignal (Alarmquelle)

- P1: erste Person
- P2: zweite Person
- P3: dritte Person
- P4: vierte Person
- P5: fünfte Person

- S1: erstes Anforderungssignal
- S2: zweites Anforderungssignal

- S1A1: Alarmsignal (erster Melder, erste Alarmquelle)
- S1A2: Alarmsignal (zweiter Melder, erste Alarmquelle)
- S1A3: Alarmsignal (dritter Melder, erste Alarmquelle)
- S1A4: Alarmsignal (vierter Melder, erste Alarmquelle)
- S1A5: Alarmsignal (fünfter Melder, erste Alarmquelle)
- S2A1: Alarmsignal (erster Melder, zweite Alarmquelle)
- S2A2: Alarmsignal (zweiter Melder, zweite Alarmquelle)
- S2A3: Alarmsignal (dritter Melder, zweite Alarmquelle)
- S2A4: Alarmsignal (vierter Melder, zweite Alarmquelle)
- S2A5: Alarmsignal (fünfter Melder, zweite Alarmquelle)
- S1C1: Countdown (erster Melder, erste Alarmquelle)
- S1C2: Countdown (zweiter Melder, erste Alarmquelle)
- S1C3: Countdown (dritter Melder, erste Alarmquelle)
- S1C4: Countdown (vierter Melder, erste Alarmquelle)
- S1C5: Countdown (fünfter Melder, erste Alarmquelle)
- S2C1: Countdown (erster Melder, zweite Alarmquelle)
- S2C2: Countdown (zweiter Melder, zweite Alarmquelle)
- S2C3: Countdown (dritter Melder, zweite Alarmquelle)
- S2C4: Countdown (vierter Melder, zweite Alarmquelle)
- S2C5: Countdown (fünfter Melder, zweite Alarmquelle)

- SHC1: Sicherheitscountdown (erste Alarmquelle)
- SHC2: Sicherheitscountdown (zweite Alarmquelle)

## Patentansprüche

1. Verfahren zur Alarmierung von Personen (P1, P2, P3, P4, P5), die sich im Bereich eines Melders (1, 2, 3, 4, 5) befinden, in Abhängigkeit von Anforderungssignalen (S1, S2) von wenigstens einer Alarmquelle (10, 11),
- wobei jeder Melder (1, 2, 3, 4, 5) aktuelle Countdowns (S1C1, S1C2, S1C3, S1C4, S1C5, S2C1, S2C2, S2C3, S2C4, S2C5) für jede der Alarmquellen (10, 11) festlegt,
- wobei bei Ausgabe eines Anforderungssignals (S1, S2) von einer der Alarmquellen (10, 11) die zugehörigen Countdowns (S1C1, S1C2, S1C3, S1C4, S1C5, S2C1, S2C2, S2C3, S2C4, S2C5) aller Melder (1, 2, 3, 4, 5) gestartet werden, die das Anforderungssignal (S1, S2) empfangen,
∘ wobei der Melder (1, 2, 3, 4, 5), dessen gestarteter Countdown (S1C1, S1C2, S1C3, S1C4, S1C5, S2C1, S2C2, S2C3, S2C4, S2C5) abläuft, ein Alarmsignal (S1A1, S1A2, S1A3, S1A4, S1A5, S2A1, S2A2, S2A3, S2A4, S2A5) ausgibt,
∘ wobei die manuelle Annahme eines ausgegebenen Alarmsignals (S1A1, S1A2, S1A3, S1A4, S1A5, S2A1, S2A2, S2A3, S2A4, S2A5) durch eine Person (P1, P2, P3, P4, P5), die sich im Bereich des Melders (1, 2, 3, 4, 5) befindet, der das Alarmsignal (S1A1, S1A2, S1A3, S1A4, S1A5, S2A1, S2A2, S2A3, S2A4, S2A5) ausgibt, die gestarteten Countdowns (S1C1, S1C2, S1C3, S1C4, S1C5, S2C1, S2C2, S2C3, S2C4, S2C5) der anderen Melder (1, 2, 3, 4, 5) abbricht.

2. Verfahren nach Anspruch 1, **dadurch gekennzeichnet, dass** wenigstens einer der Melder (1, 2, 3, 4, 5) ein tragbarer Funkmelder ist, welchen eine der Personen (P1, P2, P3, P4, P5) mit sich führt.

3. Verfahren nach einem der vorhergehenden Ansprüche, **dadurch gekennzeichnet, dass** die manuelle Ablehnung eines Alarmsignals (S1A1, S1A2, S1A3, S1A4, S1A5, S2A1, S2A2, S2A3, S2A4, S2A5) durch eine Person (P1, P2, P3, P4, P5), die sich im Bereich des Melders (1, 2, 3, 4, 5) befindet, der ein Alarmsignal (S1A1, S1A2, S1A3, S1A4, S1A5, S2A1, S2A2, S2A3, S2A4, S2A5) ausgibt, den Countdown (S1C1, S1C2, S1C3, S1C4, S1C5, S2C1, S2C2, S2C3, S2C4, S2C5) der anderen Melder (1, 2, 3, 4, 5) reduziert,

4. Verfahren nach einem der vorhergehenden Ansprüche, **dadurch gekennzeichnet, dass** der kürzeste Countdown (S1C1, S1C2, S1C3, S1C4, S1C5, S2C1, S2C2, S2C3, S2C4, S2C5) für jede Alarmquelle (10, 11) gleich null ist und der oder die diesen kürzesten Countdown (S1C1, S1C2, S1C3, S1C4, S1C5, S2C1, S2C2, S2C3, S2C4, S2C5) enthaltenden Melder (1, 2, 3, 4, 5) sofort ein Alarmsignal (S1A1, S1A2, S1A3, S1A4, S1A5, S2A1, S2A2, S2A3, S2A4, S2A5) ausgeben, wenn sie ein Anforderungssignal (S1, S2) empfangen.

5. Verfahren nach einem der vorhergehenden Ansprüche, **dadurch gekennzeichnet, dass** wenigstens zwei Alarmquellen (10, 11) vorhanden sind und jedem ausgegebenen Anforderungssignal (S1, S2) eine Kennung der auslösenden Alarmquelle (10, 11) zugefügt ist.

6. Verfahren nach einem der vorhergehenden Ansprüche, **dadurch gekennzeichnet, dass** die Alarmquellen (10, 11) und die Melder (1, 2, 3, 4, 5) jeweils ausschließlich mit einem Alarmmanagement-Server kommunizieren.

7. Verfahren nach einem der vorhergehenden Ansprüche, **dadurch gekennzeichnet, dass** von jedem Melder (1, 2, 3, 4, 5) die Position relativ zu jeder der Alarmquellen (10, 11) ermittelt und den anderen Meldern (1, 2, 3, 4, 5) bereitgestellt wird, wobei die Länge der Countdowns (S1C1, S1C2, S1C3, S1C4, S1C5, S2C1, S2C2, S2C3, S2C4, S2C5) von der eigenen Position und den Positionen der anderen Melder (1, 2, 3, 4, 5) relativ zu der Alarmquelle (10, 11) abhängt, zu der der Countdown (S1C1, S1C2, S1C3, S1C4, S1C5, S2C1, S2C2, S2C3, S2C4, S2C5) festgelegt wird.

8. Verfahren nach einem der vorhergehenden Ansprüche, **dadurch gekennzeichnet, dass** jeder Melder (1, 2, 3, 4, 5) seine Countdowns (S1C1, S1C2, S1C3, S1C4, S1C5, S2C1, S2C2, S2C3, S2C4, S2C5) unter Berücksichtigung der absoluten Entfernungen oder räumlichen Wegentfernung des Melders (1, 2, 3, 4, 5) und den anderen Meldern (1, 2, 3, 4, 5) zum Einsatzort der zugehörigen Alarmquelle (10, 11) festlegt.

9. Verfahren nach einem der vorhergehenden Ansprüche, **dadurch gekennzeichnet, dass** jeder Countdown (S1C1, S1C2, S1C3, S1C4, S1C5, S2C1, S2C2, S2C3, S2C4, S2C5) in Echtzeit oder getaktet aktualisiert wird.

10. Verfahren nach einem der vorhergehenden Ansprüche, **dadurch gekennzeichnet, dass** jeder Countdown (S1C1, S1C2, S1C3, S1C4, S1C5, S2C1, S2C2, S2C3, S2C4, S2C5) unter Berücksichtigung des Melders (1, 2, 3, 4, 5), insbesondere unter Berücksichtigung der dem Melder (1, 2, 3, 4, 5) zugeordneten Personen (P1, P2, P3, P4, P5), festgelegt wird.

11. Verfahren nach einem der vorhergehenden Ansprüche, **dadurch gekennzeichnet, dass** durch eine Person eine fehlende Verfügbarkeit am Melder (1, 2, 3, 4, 5) eingebbar ist, die bei der Festlegung, insbesondere bei der Aktualisierung, eines jeden Countdowns (S1C1, S1C2, S1C3, S1C4, S1C5, S2C1, S2C2, S2C3, S2C4, S2C5) berücksichtigt wird.

12. Verfahren nach einem der vorhergehenden Ansprüche, **dadurch gekennzeichnet, dass** ein Melder (1, 2, 3, 4, 5) nach Annahme eines Alarmsignals (S1A1, S1A2, S1A3, S1A4, S1A5, S2A1, S2A2, S2A3, S2A4, S2A5) für einen definierten Zeitraum als nicht verfügbar gilt, was bei der Festlegung, insbesondere bei der Aktualisierung, eines jeden Countdowns (S1C1, S1C2, S1C3, S1C4, S1C5, S2C1, S2C2, S2C3, S2C4, S2C5) berücksichtigt wird.

13. Verfahren nach einem der vorhergehenden Ansprüche, **dadurch gekennzeichnet, dass** die Alarmquelle (10, 11) bei Ausgabe eines Anforderungssignals (S1, S2) einen Sicherheitscountdown (SHC1, SHC2) startet, der länger ist als die mit dem Anforderungssignal (S1, S2) gestarteten Countdowns (S1C1, S1C2, S1C3, S1C4, S1C5, S2C1, S2C2, S2C3, S2C4, S2C5) der Melder (1, 2, 3, 4, 5), wobei bei Ablauf des Sicherheitscountdowns (SHC1, SHC2) von der Alarmquelle (10, 11) wieder ein Anforderungssignal (S1, S2) ausgegeben wird oder ein eigenes akustisches und/oder visuelles und/oder taktiles Alarmsignal (NS1, NS2) ausgegeben wird.

14. Verfahren nach einem der vorhergehenden Ansprüche, **dadurch gekennzeichnet, dass** festlegbar ist, welche der Melder (1, 2, 3, 4, 5) welcher der Alarmquellen (10, 11) zugeordnet sind, und dies bei der Festlegung der Countdowns (S1C1, S1C2, S1C3, S1C4, S1C5, S2C1, S2C2, S2C3, S2C4, S2C5) berücksichtigt wird.

## Claims

1. Method for alerting persons (P1, P2, P3, P4, P5) in the region of a detector (1, 2, 3, 4, 5) on the basis of request signals (S1, S2) from at least one alarm source (10, 11),
- wherein each detector (1, 2, 3, 4, 5) determines current countdowns (S1C1, S1C2, S1C3, S1C4, S1C5, S2C1, S2C2, S2C3, S2C4, S2C5) for each of the alarm sources (10, 11),
- wherein, if a request signal (S1, S2) is output by one of the alarm sources (10, 11), the associated countdowns (S1C1, S1C2, S1C3, S1C4, S1C5, S2C1, S2C2, S2C3, S2C4, S2C5) of all detectors (1, 2, 3, 4, 5) which receive the request signal (S1, S2) are started,
∘ wherein the detector (1, 2, 3, 4, 5) whose started countdown (S1C1, S1C2, S1C3, S1C4, S1C5, S2C1, S2C2, S2C3, S2C4, S2C5) expires outputs an alarm signal (S1A1, S1A2, S1A3, S1A4, S1A5, S2A1, S2A2, S2A3, S2A4, S2A5),
∘ wherein the manual acceptance of an output alarm signal (S1A1, S1A2, S1A3, S1A4, S1A5, S2A1, S2A2, S2A3, S2A4, S2A5) by a person (P1, P2, P3, P4, P5) in the region of the detector (1, 2, 3, 4, 5) which outputs the alarm signal (S1A1, S1A2, S1A3, S1A4, S1A5, S2A1, S2A2, S2A3, S2A4, S2A5) aborts the started countdowns (S1C1, S1C2, S1C3, S1C4, S1C5, S2C1, S2C2, S2C3, S2C4, S2C5) of the other detectors (1, 2, 3, 4, 5).

2. Method according to Claim 1, **characterized in that** at least one of the detectors (1, 2, 3, 4, 5) is a portable radio detector carried by one of the persons (P1, P2, P3, P4, P5).

3. Method according to one of the preceding claims, **characterized in that** the manual rejection of an alarm signal (S1A1, S1A2, S1A3, S1A4, S1A5, S2A1, S2A2, S2A3, S2A4, S2A5) by a person (P1, P2, P3, P4, P5) in the region of the detector (1, 2, 3, 4, 5) which outputs an alarm signal (S1A1, S1A2, S1A3, S1A4, S1A5, S2A1, S2A2, S2A3, S2A4, S2A5) reduces the countdown (S1C1, S1C2, S1C3, S1C4, S1C5, S2C1, S2C2, S2C3, S2C4, S2C5) of the other detectors (1, 2, 3, 4, 5).

4. Method according to one of the preceding claims, **characterized in that** the shortest countdown (S1C1, S1C2, S1C3, S1C4, S1C5, S2C1, S2C2, S2C3, S2C4, S2C5) for each alarm source (10, 11) is equal to zero and the detector(s) (1, 2, 3, 4, 5) containing this shortest countdown (S1C1, S1C2, S1C3, S1C4, S1C5, S2C1, S2C2, S2C3, S2C4, S2C5) immediately output(s) an alarm signal (S1A1, S1A2, S1A3, S1A4, S1A5, S2A1, S2A2, S2A3, S2A4, S2A5) if it/they receive(s) a request signal (S1, S2).

5. Method according to one of the preceding claims, **characterized in that** at least two alarm sources (10, 11) are present and an identifier of the triggering alarm source (10, 11) is added to each request signal (S1, S2) which is output.

6. Method according to one of the preceding claims, **characterized in that** the alarm sources (10, 11) and the detectors (1, 2, 3, 4, 5) each communicate solely with an alarm management server.

7. Method according to one of the preceding claims, **characterized in that** the position relative to each of the alarm sources (10, 11) is determined by each detector (1, 2, 3, 4, 5) and is made available to the other detectors (1, 2, 3, 4, 5), wherein the length of the countdowns (S1C1, S1C2, S1C3, S1C4, S1C5, S2C1, S2C2, S2C3, S2C4, S2C5) depends on the detector's own position and the positions of the other detectors (1, 2, 3, 4, 5) relative to the alarm source (10, 11) for which the countdown (S1C1, S1C2, S1C3, S1C4, S1C5, S2C1, S2C2, S2C3, S2C4, S2C5) is determined.

8. Method according to one of the preceding claims, **characterized in that** each detector (1, 2, 3, 4, 5) determines its countdowns (S1C1, S1C2, S1C3, S1C4, S1C5, S2C1, S2C2, S2C3, S2C4, S2C5) taking into account the absolute distances or spatial walking distance of the detector (1, 2, 3, 4, 5) and the other detectors (1, 2, 3, 4, 5) relative to the usage location of the associated alarm source (10, 11).

9. Method according to one of the preceding claims, **characterized in that** each countdown (S1C1, S1C2, S1C3, S1C4, S1C5, S2C1, S2C2, S2C3, S2C4, S2C5) is updated in real time or in a clocked manner.

10. Method according to one of the preceding claims, **characterized in that** each countdown (S1C1, S1C2, S1C3, S1C4, S1C5, S2C1, S2C2, S2C3, S2C4, S2C5) is determined taking into account the detector (1, 2, 3, 4, 5), in particular taking into account the persons (P1, P2, P3, P4, P5) assigned to the detector (1, 2, 3, 4, 5).

11. Method according to one of the preceding claims, **characterized in that** a lack of availability can be input by a person at the detector (1, 2, 3, 4, 5), which lack of availability is taken into account when determining, in particular when updating, each countdown (S1C1, S1C2, S1C3, S1C4, S1C5, S2C1, S2C2, S2C3, S2C4, S2C5).

12. Method according to one of the preceding claims, **characterized in that,** after the acceptance of an alarm signal (S1A1, S1A2, S1A3, S1A4, S1A5, S2A1, S2A2, S2A3, S2A4, S2A5), a detector (1, 2, 3, 4, 5) is considered to be unavailable for a defined period, which is taken into account when determining, in particular when updating, each countdown (S1C1, S1C2, S1C3, S1C4, S1C5, S2C1, S2C2, S2C3, S2C4, S2C5).

13. Method according to one of the preceding claims, **characterized in that,** when a request signal (S1, S2) is output, the alarm source (10, 11) starts a safety countdown (SHC1, SHC2) which is longer than the countdowns (S1C1, S1C2, S1C3, S1C4, S1C5, S2C1, S2C2, S2C3, S2C4, S2C5) of the detectors (1, 2, 3, 4, 5) started with the request signal (S1, S2), wherein, when the safety countdown (SHC1, SHC2) expires, a request signal (S1, S2) is output by the alarm source (10, 11) again or a separate acoustic and/or visual and/or tactile alarm signal (NS1, NS2) is output.

14. Method according to one of the preceding claims, **characterized in that** it is possible to determine which of the detectors (1, 2, 3, 4, 5) are assigned to which of the alarm sources (10, 11) and this is taken into account when determining the countdowns (S1C1, S1C2, S1C3, S1C4, S1C5, S2C1, S2C2, S2C3, S2C4, S2C5).

## Revendications

1. Procédé d'alerte de personnes (P1, P2, P3, P4, P5), qui se trouvent dans la zone d'un avertisseur (1, 2, 3, 4, 5), en fonction de signaux de demande (S1, S2) d'au moins une source d'alarme (10, 11),
- sachant que chaque avertisseur (1, 2, 3, 4, 5) spécifie des comptes à rebours actuels (S1C1, S1C2, S1C3, S1C4, S1C5, S2C1, S2C2, S2C3, S2C4, S2C5) pour chacune des sources d'alarme (10, 11),
- sachant que, lorsqu'un signal de demande (S1, S2) est délivré par une des sources d'alarme (10, 11), les comptes à rebours associés (S1C1, S1C2, S1C3, S1C4, S1C5, S2C1, S2C2, S2C3, S2C4, S2C5) de tous les avertisseurs (1, 2, 3, 4, 5) qui reçoivent le signal de demande (S1, S2) sont démarrés,
- sachant que l'avertisseur (1, 2, 3, 4, 5) dont le compte à rebours démarré (S1C1, S1C2, S1C3, S1C4, S1C5, S2C1, S2C2, S2C3, S2C4, S2C5) expire délivre un signal d'alarme (S1A1, S1A2, S1A3, S1A4, S1A5, S2A1, S2A2, S2A3, S2A4, S2A5),
- sachant que l'acceptation manuelle d'un signal d'alarme délivré (S1A1, S1A2, S1A3, S1A4, S1A5, S2A1, S2A2, S2A3, S2A4, S2A5), par une personne (P1, P2, P3, P4, P5) qui se trouve dans la zone de l'avertisseur (1, 2, 3, 4, 5) qui délivre le signal d'alarme (S1A1, S1A2, S1A3, S1A4, S1A5, S2A1, S2A2, S2A3, S2A4, S2A5), annule les comptes à rebours démarrés (S1C1, S1C2, S1C3, S1C4, S1C5, S2C1, S2C2, S2C3, S2C4, S2C5) des autres avertisseurs (1, 2, 3, 4, 5).

2. Procédé selon la revendication 1, **caractérisé en ce qu'**au moins un des avertisseurs (1, 2, 3, 4, 5) est un avertisseur sans fil portable, qu'une des personnes (P1, P2, P3, P4, P5) emporte avec elle.

3. Procédé selon l'une des revendications précédentes, **caractérisé en ce que** le rejet manuel d'un signal d'alarme (S1A1, S1A2, S1A3, S1A4, S1A5, S2A1, S2A2, S2A3, S2A4, S2A5), par une personne (P1, P2, P3, P4, P5) qui se trouve dans la zone de l'avertisseur (1, 2, 3, 4, 5) qui délivre un signal d'alarme (S1A1, S1A2, S1A3, S1A4, S1A5, S2A1, S2A2, S2A3, S2A4, S2A5), réduit le compte à rebours (S1C1, S1C2, S1C3, S1C4, S1C5, S2C1, S2C2, S2C3, S2C4, S2C5) des autres avertisseurs (1, 2, 3, 4, 5).

4. Procédé selon l'une des revendications précédentes, **caractérisé en ce que** le compte à rebours le plus court (S1C1, S1C2, S1C3, S1C4, S1C5, S2C1, S2C2, S2C3, S2C4, S2C5) pour chaque source d'alarme (10, 11) est égal à zéro, et le ou les avertisseurs (1, 2, 3, 4, 5) contenant ce compte à rebours le plus court (S1C1, S1C2, S1C3, S1C4, S1C5, S2C1, S2C2, S2C3, S2C4, S2C5) délivrent immédiatement un signal d'alarme (S1A1, S1A2, S1A3, S1A4, S1A5, S2A1, S2A2, S2A3, S2A4, S2A5) lorsqu'ils reçoivent un signal de demande (S1, S2).

5. Procédé selon l'une des revendications précédentes, **caractérisé en ce qu'**au moins deux sources d'alarme (10, 11) sont présentes, et une identification de la source d'alarme déclencheuse (10, 11) est ajoutée à chaque signal de demande délivré (S1, S2).

6. Procédé selon l'une des revendications précédentes, **caractérisé en ce que** les sources d'alarme (10, 11) et les avertisseurs (1, 2, 3, 4, 5) communiquent respectivement exclusivement avec un serveur de gestion d'alarme.

7. Procédé selon l'une des revendications précédentes, **caractérisé en ce que** chaque avertisseur (1, 2, 3, 4, 5) détermine la position par rapport à chacune des sources d'alarme (10, 11) et la fournit aux autres avertisseurs (1, 2, 3, 4, 5), sachant que la longueur du compte à rebours (S1C1, S1C2, S1C3, S1C4, S1C5, S2C1, S2C2, S2C3, S2C4, S2C5) dépend de la propre position et des positions des autres avertisseurs (1, 2, 3, 4, 5) par rapport à la source d'alarme (10, 11) pour laquelle le compte à rebours (S1C1, S1C2, S1C3, S1C4, S1C5, S2C1, S2C2, S2C3, S2C4, S2C5) est spécifié.

8. Procédé selon l'une des revendications précédentes, **caractérisé en ce que** chaque avertisseur (1, 2, 3, 4, 5) spécifie ses comptes à rebours (S1C1, S1C2, S1C3, S1C4, S1C5, S2C1, S2C2, S2C3, S2C4, S2C5) en tenant compte des distances absolues ou des distances de parcours dans l'espace de l'avertisseur (1, 2, 3, 4, 5) et des autres avertisseurs (1, 2, 3, 4, 5) par rapport au lieu d'utilisation de la source d'alarme correspondante (10, 11).

9. Procédé selon l'une des revendications précédentes, **caractérisé en ce que** chaque compte à rebours (S1C1, S1C2, S1C3, S1C4, S1C5, S2C1, S2C2, S2C3, S2C4, S2C5) est actualisé en temps réel ou cycliquement.

10. Procédé selon l'une des revendications précédentes, **caractérisé en ce que** chaque compte à rebours (S1C1, S1C2, S1C3, S1C4, S1C5, S2C1, S2C2, S2C3, S2C4, S2C5) est spécifié en tenant compte de l'avertisseur (1, 2, 3, 4, 5), en particulier en tenant compte des personnes (P1, P2, P3, P4, P5) associées à l'avertisseur (1, 2, 3, 4, 5).

11. Procédé selon l'une des revendications précédentes, **caractérisé en ce qu'**une personne peut saisir sur l'avertisseur (1, 2, 3, 4, 5) une absence de disponibilité qui est prise en compte lors de la spécification, en particulier lors de l'actualisation, de chaque compte à rebours (S1C1, S1C2, S1C3, S1C4, S1C5, S2C1, S2C2, S2C3, S2C4, S2C5).

12. Procédé selon l'une des revendications précédentes, **caractérisé en ce qu'**un avertisseur (1, 2, 3, 4, 5), à la suite de l'acceptation d'un signal d'alarme (S1A1, S1A2, S1A3, S1A4, S1A5, S2A1, S2A2, S2A3, S2A4, S2A5), est considéré comme non disponible pour une durée définie, ce qui est pris en compte lors de la spécification, en particulier lors de l'actualisation, de chaque compte à rebours (S1C1, S1C2, S1C3, S1C4, S1C5, S2C1, S2C2, S2C3, S2C4, S2C5).

13. Procédé selon l'une des revendications précédentes, **caractérisé en ce que** la source d'alarme (10, 11), lors de la délivrance d'un signal de demande (S1, S2), démarre un compte à rebours de sécurité (SHC1, SHC2) qui est plus long que les comptes à rebours (S1C1, S1C2, S1C3, S1C4, S1C5, S2C1, S2C2, S2C3, S2C4, S2C5) des avertisseurs (1, 2, 3, 4, 5) qui sont démarrés avec le signal de demande (S1, S2), sachant qu'à l'expiration du compte à rebours de sécurité (SHC1, SHC2), la source d'alarme (10, 11) délivre à nouveau un signal de demande (S1, S2) ou délivre un signal d'alarme distinct (NS1, NS2) acoustique et/ou visuel et/ou tactile.

14. Procédé selon l'une des revendications précédentes, **caractérisé en ce qu'**on peut spécifier lesquels des avertisseurs (1, 2, 3, 4, 5) sont associés auxquelles des sources d'alarme (10, 11), et cela est pris en compte lors de la spécification des comptes à rebours (S1C1, S1C2, S1C3, S1C4, S1C5, S2C1, S2C2, S2C3, S2C4, S2C5).
